## Europäisches Patentamt
## European Patent Office
## Office européen des brevets

(19)

(11) Numéro de publication: **0 141 768**
**B1**

(12) ## FASCICULE DE BREVET EUROPÉEN

(45) Date de publication du fascicule du brevet:
**25.01.89**

(51) Int. Cl.⁴: **A 61 K 9/50**

(21) Numéro de dépôt: **84430034.3**

(22) Date de dépôt: **05.10.84**

(54) **Procédé perfectionné d'obtention de liposomes unilamellaires de diamètres élevés, leur application pharmacologique pour l'encapsulage d'un principe actif en vue de son administration extemporanée et dispositif correspondant.**

(30) Priorité: **06.10.83 FR 8316239**

(43) Date de publication de la demande:
**15.05.85 Bulletin 85/20**

(45) Mention de la délivrance du brevet:
**25.01.89 Bulletin 89/4**

(84) Etats contractants désignés:
**AT BE CH DE GB IT LI LU NL SE**

(73) Titulaire: **CENTRE NATIONAL DE LA RECHERCHE SCIENTIFIQUE (CNRS), 15, Quai Anatole France, F-75007 Paris (FR)**
Titulaire: **INSTITUT NATIONAL DE LA SANTE ET DE LA RECHERCHE MEDICALE (INSERM), 101, rue de Tolbiac, F-75654 Paris Cédex 13 (FR)**

(72) Inventeur: **Philippot, Jean, Avenue des Génévriers, F-34980 St Clement La Riviere (FR)**
Inventeur: **Liautard, Jean- Pierre, 481 rue Croix de las Casas, F-34100 Montpellier (FR)**

(74) Mandataire: **Rinuy, Santarelli, 14, avenue de la Grande Armée, F-75017 Paris (FR)**

(56) Documents cité:
EP-A-0 032 578
EP-A-0 056 781
WO-A-81/03280
FR-A-2 441 385
FR-A-2 446 635

CHEMICAL ABSTRACTS, vol. 99, no. 25, 19 décembre 1983, page 354, no. 209201e, Columbus, Ohio, USA; J. PHILIPPOT et al.: "A very mild method allowing the encapsulation of very high amounts of macromolecules into very large (1000-nm) unilamellar liposomes" & BIOCHIM. BIOPHYS. ACTA 1983, 734(2), 137-14
BIOCHEMISTRY, vol. 18, no. 19, 18 septembre 1979, pages 4173-4176, American Chemical Society, USA; V. RHODEN et al.: "Formation of unilamellar lipid vesicles of controllable dimensions by detergent dialysis"
CHEMICAL ABSTRACTS, vol. 95, no. 9, 31 août 1981, page 393, no. 76456k, Columbus, Ohio, USA; R.A. SCHWENDENER et al.: "n-Alkylglucosides as detergents for the preparation of highly homogeneous bilayer liposomes of variable sizes (60-240 nm diam.) applying defined rates of

(56) Documents cité: (suite)
detergent removal by dialysis"
BIOCHEMISTRY; vol. 20, no. 4, 17 février 1981, pages 833-840, American Chemical Society, USA; L.T. MIMMS et al.: "Phospholipid vesicle formation and transmembrane protein incorporation using octyl glucoside"
CHEMICAL ABSTRACTS, vol. 98, no. 1, 3 janvier 1983, page 277, no. 2483g, Columbus, Ohio, USA; K.KRETSCHMER et al.: "A simple method to measure critical micellar concentrations and to follow detergent removal during liposome preparation"
BIOCHEMISTRY, vol. 19, 19 août 1980, pages 4108-4115, American Chemical Society, USA; P. ROSEVEAR et al.: "Alkyl glycoside detergents: A simpler synthesis and their effects on kinetic and physical properties of cytochrome C oxidase"
CHEMICAL ABSTRACTS, vol. 97, no. 5, 2 août 1982, page 216, no. 35020m, Columbus, Ohio, USA; A. ALONSO et al.: "Increase in size of sonicated phospholipid vesicles in the presence of

**Description**

La présente invention concerne un procédé perfectionné pour l'obtention de liposomes unilamellaires de diamètres élevés et leur application pharmacologique pour l'encapsulage d'un principe actif en vue de son administration extemporanée.

Au cours de ces dernières années, de nombreuses études ont montré que les lipsomes peuvent être utilisés peur l'encapsulage et le transfert d'une substance active dans les cellules. A cet effet, des vésicules de phospholipide ont principalement été préparées par des techniques physiques comme les ultra-sons, la technique dite de "French press" ou des techniques chimiques, en utilisant des solvants organiques ou des détergents, suivies de l'élimination de ces derniers.

Or, les liposomes obtenus à l'heure actuelle présentent, soit des structures, soit des dimensions (diamètres de l'ordre de 200 nm ou moins (voir Mimms et coll. Biochemistry, 1981, (20), pp 833 - 840, EP-A.O N° 056 781 et Kretschmer Chemical Abstracts vol. 98 N°1, 3 janvier 1983 page 277)) ne permettant pas toujours, soit d'encapsuler certaines macromolécules présentant un certain encombrement, soit d'encapsuler une quantité relativement importante de substance active, les quantités encapsulées jusqu'à présent restant limitées à certaines valeurs.

Le demandeurs se sont par conséquent fixé comme objectif d'obtenir de gros liposomes unilamellaires de diamètres nettement supérieurs à 200 nm et qui soient, d'une part, capables d'encapsuler des macromolécules de dimensions importances (protéines ou acides nucléiques), et d'autre part, capables d'encapsuler des quantités d'une substance active plus importantes qu'il n'était possible jusqu'à présent. Par ailleurs, le procédé d'obtention de ces liposomes devrait être suffisamment modéré et plus spécialement devrait éviter le contact avec un solvant organique dénaturant les protéines ou des énergies élevées telles que l'émssion d'ultra-sons ou l'application de pressions susceptibles de briser les molécules d'acides nucléiques. La technique faisant appel à un détergent semble être la meilleure façon de préserver la structure et l'activité de ces macromolécules. Toutefois, par cette technique, non seulement on ne dépasse pas des diamètres de l'ordre de 200 nm comme c'est le cas par exemple en utilisant le procédé décrit par Rhoden et coll. dans Biochemistry vol. 18, n° 19, 18 septembre 1979 pages 4173 - 4176, mais sa mise en oeuvre nécessite plusieurs heures (de 2 à 10 heures comme c'est le cas dans le procédé décrit dans le brevet EP-A-O N° 032 578).

L'élimination du détergent par dialyse ou par la technique de filtration sur gel fournit des liposomes unilamellaires. Le volume interne de ces liposomes varie avec la nature du détergent, le rapport claire du détergent aux phospholipides, la composition en lipides des vésicules et la vitesse de dialyse. La dimensions des vésicules s'est avérée être différente en taille mais du même ordre de grandeur, que ce soit après dialyse ou après filtration sur gel. Toutefois, la rareté et/ou le coût de la molécule à encapsuler (anticorps, enzymes, gènes purifiés ou porteurs de ARN) nécessite un taux d'encapsulation très élevé et exclut les techniques exigeant de grandes quantités de substance à encapsuler comme celle de l'élimination du détergent par filtration sur gel.

Le procédé selon la présente invention est fondé sur la technique de l'élimination du détergent par dialyse et en constitue un perfectionnement. En effet, un des principaux inconvénients que l'on rencontre lors de la formation de liposomes par la technique mettant en oeuvre des détergents réside dans le fait que l'élimination du détergent n'est jamais totale; il reste une quantité très souvent non négligeable de détergent, même après une dialyse poussée. C'est ainsi qu'avec les détergents habituellement utilisés, en particulier le cholate ou le desoxycholate de sodium et le "Triton X 100'"®, la quantité résiduelle peut aller de quelques % à 10 %.

Or, les demandeurs ont trouvé de façon imprévisible que si l'on utilise comme détergent pour la solubilisation de lipides, en vue de la formation d'un liposome, un détergent neutre présentant une concentration critique micellaire élevée, sous certaines conditions de dialyse, son élimination rapide et quasiment totale peut être obtenue, permettant ainsi de réaliser des liposomes encapsulant un principe actif tel qu'un médicament pratiquement exempt de détergent.

Par ailleurs, on obtient des liposomes unilamellaires de diamètre élevé pouvant aller de 200 à 1 000 nm et donc capables d'encapsuler, soit de grosses molécules en quantités appropriées, soit des quantités importantes de molécules moins encombrantes palliant ainsi les inconvénients inhérents aux liposomes connus.

Selon l'invention, il est proposé un procédé perfectionné pour l'obtention de liposomes unilamallaires de diamètres élevés supérieurs à 200 nm comprenant la solubilisation de lipides au moyen d'un détergent neutre, puis l'éliminaion dudit détergent par tout moyen approprié, procédé caractérisé par le fait que ledit détergent neutre présente une concentration critique micellaire élevée, qu'on utilise un rapport détergent: lipide de 10 : 1 à 12 : 1 et que l'éliminaion de ce détergent est effectuée au moyen de billes de polymères à pouvoir adsorbant élevé du type styrène-divinyl-benzène.

En vue de l'application particulière en tant que véhicule de médicament pratiquement exempt de détergent, les demandeurs ont constaté que, comme détergent répondant à la définition ci-dessus convenant selon l'invention, on peut citer l'octylglucoside qui présente en outre la propriété d'être compatible avec une grande quantité des substances actives que l'on viendrait à encapsuler.

Il a été de plus établi par les demandeurs que l'élimination du détergent est d'autant plus totale et rapide que le rapport détergent/billes (µmole/mg) est faible.

L'invention couvre également le procédé ci-dessus, appliqué à l'encapsulage d'une substance active, caractérisé par le fait que l'on ajoute aux lipides désirés, solubilisés dans le détergent conforme à l'invention, la substance à encapsuler et que l'on élimine ledit détergent au moyen desdites billes de polymères de synthèse à pouvoir adsorbant élevé.

Elle couvre également la préparation extemporanée d'un médicament, consistant à prélever le volume désiré d'un mélange de la solution mixte de lipides, de détergent et de la substance à encapsuler, à procéder à l'élimination du détergent avantageusement après contact direct avec les billes, puis à injecter au patient le liposome exempt de détergent.

Suivant un mode de réalisation avantageux, au moment de l'emploi, on opère au moyen d'une seringue de prélèvement chargée de l'agent adsorbant le détergent et munie à sa base d'un filtre ou tamis approprié.

D'autres avantages et les caractéristiques de l'invention ressortiront plus clairement de la description qui va suivre.

Comme indiqué précédemment, l'une des principales difficultés rencontrées lors de la formation des liposomes par la technique faisant appel à un détergent solubilisant réside dans le fait qu'une quantité résiduelle non négligeable de détergent subsiste dans le liposome obtenu même après avoir eu recours, pour l'élimination dudit détergent, à une opération de dialyse poussée (quelques pour cent dans le cas du cholate ou du desoxycholate et environ 10 % dans le cas du Triton X-100®). Or, si l'on choisit par exemple de l'octylglucoside comme détergent, on constate qu'il peut être éliminé presque complètement (quantité résiduelle inférieure à 0,05 %) et rapidement. On attribue ce résultat inattendu et imprévisible, et c'est ce qui constitue le fondement de l'invention et en fait son intérêt, au fait que ce détergent présente une concentration micellaire très élevée qui facilite son rejet rapide de complexes micellaires mixtes. On a constaté aussi de façon assez générale qu'un rapport molaire d'un tel détergent lipide compris entre 10 : 1 et 12 : 1 convenait pour obtenir une solution limpide de lipide. On notera que la présence de cholestérol ne modifie pas le degré de solubilisation de mélanges de lipides.

Pour ce qui est de l'élimination de ce détergent, on a utilisé la dialyse et un polymère de haut pouvoir adsorbant avantageusement sous forme de billes, ce polymère étant choisi de préférence parmi ceux formés de styrène-divinylbenzène du type "Amberlite X® AD-2", commercialisées par la Société BIO RAD sous le nom de "Bio Beads® SM2".

Des essais effectués avec ce type de billes mais avec, comme détergent, outre l'octylglucoside, le "TRITON X-100" et le desoxycholate de sodium, montrent que l'adsorption du détergent et donc de l'octylglucoside était fonction du rapport détergent/billes. De plus, comparativement à l'adsorption correspondante du TRITON X-100®, on note que l'octylglucoside se fixe plus rapidement sur ces billes que le TRITON X-100®. Par ailleurs, on observe qu'il existe un rapport détergent/billes optimum au-delà duquel l'élimination du détergent n'est plus améliorée. Ce rapport optimum est de "0,11" pour l'octylglucoside (1 µmole de détergent/9 mg de billes) et il correspond à un rejet total du détergent du milieu, alors que ce même rapport optimum est de "0,05" dans le cas du TRITON X-100® (1 µmole détergent/20 mg de billes) avec une quantité résiduelle de 0,25 % de détergent, quantité que l'on ne peut pas réduire en ajoutant une quantité supplémentaire de billes (29 mg de billes/µmole de détergent). Ceci confirme ce que tout homme de l'art sait de la difficulté qu'il y a à éliminer le TRITON X-100® de tels milieux. Toutefois, ceci montre le net perfectionnement apporté lorsque l'on procède avec les moyens de l'invention.

Une autre série d'essais a permis aussi d'apprécier la capacité de liaison desdites billes avec les phospholipides au cours d'une préparation typique de liposomes en utilisant le contact direct entre des micelles mixtes détergent-phospholipide et des billes telles que définies ci-dessus, et de comparer aussi les effets du TRITON X-100® et de l'octylglucoside sur ce phénomène. A cet effet, on a solubilisé une quantité identique de lipides (rapport molaire PC/PS de 1 : 1 - PC désignant la phosphatidylcholine et PS la phosphatidylserine) au moyen de ces détergents avec un rapport molaire détergent/phospholipides de 8 puis on a mélangé des échantillons de ces milieux avec lesdites billes et on a déterminé par voie d'analyse cinétique les concentrations des solutions. Dans ces conditions, on peut obtenir des liposomes dans un très court laps de temps. Toutefois, on constate qu'une importante quantité de lipides semble se fixer aux billes dans les deux cas. En fait, il ne s'agit que d'une dilution due au volume de liquide emprisonné par les billes. Donc il n'y a pas de perte de lipide par fixation. En tout état de cause, on constate qu'au delà d'un certain seuil de concentration en billes, cette élimination totale et rapide de ce détergent est obtenue quelle que soit la quantité de billes utilisée. Cette élimination est cependant d'autant plus rapide que le rapport détergent/billes (µmole/mg) est faible. Par ailleurs, la quantité de lipide présente dans le mélange (telle qu'elle peut être déterminée à l'aide de 14C-PC) semble décroître lorsqu'augmente la quantité de billes dans le milieu (dilution des lipides par le volume interne des billes).

Ces résultats mettent de toute façon bien en lumière l'aptitude des billes en cause à adsorber de nombreux composés différents, que leurs molécules soient neutres ou chargées. Cette aptitude des billes de polystyrène n'avait pas été signalée jusqu'à présent. Toutefois, cette technique impliquant un contact direct avec les billes peut ne pas convenir pour l'encapsulage de certaines macromolécules.

Or de tels milieux et de telles billes peuvent avantageusement être utilisés pour un tel encapsulage et ce, conformément à l'invention, en procédant par la technique de dialyse.

C'est ainsi par exemple, qu'au cours d'une série d'essais, on a procédé à l'hydratation de 20 - 30 µmoles de lipides séchés, soit au moyen de 0,125 ml d'une solution tamponnée d'anticorps fluorescent (1,25 mg de protéine), soit au moyen d'une solution de tARN (400 µg/ml) puis à leur solubilisation au moyen d'octylglucoside en utilisant un rapport molaire détergent/lipide de 10 : 1, dans un volume final de 0,675 ml. On a procédé à la dialyse contre 100 ml de solution tampon contenant des quantités différentes de billes (0,04 à 0,1 µmole de détergent/mg de billes). On a alors comparé les résultats obtenus par cette technique avec ceux obtenus par dialyse classique en changeant quatre fois un volume de 1 000 ml de solution tampon.

Pour un rapport de détergent/billes inférieur à 0,1, toutes les molécules de détergent sont adsorbées en moins d'une heure par contact direct entre le détergent et les billes. On obtient les mêmes résultats 24 heures plus tard lorsqu'on ajoute les billes au milieu de dialyse. On constate qu'un tel rapport inférieur à 0,1 accélère l'élimination du détergent. L'essai comparatif montre ainsi que cette technique donne une élimination de détergent plus rapide avec 100 ml seulement de solution tampon.

On a vérifié aussi que cette dialyse utilisant ces billes ne modifie pas la concentration en lipides.

Par ailleurs, on a mesuré, par microscopie électronique, les diamètres des vésicules obtenues. On constate que les liposomes en question présentent une répartition dimensionnelle très homogène.

L'exemple ci-après illustre un type de liposome que l'on peut obtenir en mettant en oeuvre l'invention.

**Exemple:**

1. <u>Préparation du mélange lipide-octylglucoside:</u>

On mélange dans différentes proportions les lipides (stockés à - 30°C dans un mélange 2 : 1 (v/v) de chloroforme et de méthanol) et on ajoute si on le désire, pour les besoins de l'expérimentation, une quantité appropriée de phosphatidylcholine [C14] comme élément traceur. On évapore le solvant en soumettant le mélange (concentration finale 10 - 30 mM) à un courant d'azote puis sous vide pendant une heure. On ajoute ensuite la substance à encapsuler avec une solution tampon (L) [10 mM Hepes (acide 4-(2-hydroxyéthyl)-1-piperazineéthanesulfonique, (pH 7,4) / 1 mM EGTA / 150 mM NaCl] et après agitation au Vortex, les lipides sont hydratés 30 minutes à une température supérieure à la température de transition la plus élevée des constituants du mélange. On ajoute alors le détergent (octylglucoside) avec, si nécessaire, une quantité appropriée du même détergent radioactif comme traceur pour les besoins de l'expérimentation. La suspension des liposomes multilamellaires se transforme en une solution limpide. Un tel mélange, scellé sous vide dans une ampoule, devrait pouvoir se conserver plusieurs mois.

2. <u>Elimination du détergent et formation des liposomes unilamellaires encapsulant la substance active</u>

2.1. Elimination par dialyse:

On prélève le volume désiré de la solution mixte ci-dessus (lipide + substance active + détergent) et on le dialyse contre 100 ml de tampon contenant une quantité de billes telles que définies précédemment et dans le rapport optimum. On agite quelques heures: il se forme des liposomes unilamellaires contenant, encapsulée en leur sein, la substance active ajoutée. Ces liposomes sont alors prêts à l'emploi.

Le tableau ci-dessous illustre quelques caractéristiques de types de liposomes obtenus conformément à l'invention avec élimination par dialyse.

| Propriétés | Composition de lipides | | |
|---|---|---|---|
| | PC | PC/PS (1 : 1) | PC/PS/Cholestérol (1 : 1 : 1) |
| Protéine encapsulée % | 26 | 11 ± 2 | 47 ± 4 |
| ARN encapsulé % | non déterminé | non déterminé | 45 ± 4 |
| Diamètre calculé * (nm) | 540 | 237 ± 50 | 920 ± 100 |
| Diamètre mesuré par microscopie électro-nique (nm) | 300-500 | 100-240 | 600-1100 |
| Volume interne (1/mole PL) | 20 | 8 | 35 |

*Le volume total encapsulé a été calculé sur la base d'une surface totale de 75 Å$^2$ par molécule de phospholide, par l'équation:

$$\% \text{ d'encapsulation} = 37 \times M \times d \times 10^2$$

où M concentration des phospholipides (mol/litre)
et = d = diamètre (µm)

Comme on peut le voir à l'examen de ce tableau, le diamètre des liposomes varie dans une large mesure avec la composition des lipides. Les liposomes PC-PS ont un diamètre de 200 nm, alors que l'addition de cholestérol donne des vésicules présentant un diamètre de 1.000 nm. Cet effet du cholestérol sur la dimension des vésicules est connu.

L'examen au microscope électronique a montré que ces vésicules sont unilamellaires. Ceci est confirmé par la comparaison du volume du compartiment intravésiculaire (donné par l'équation ci-dessus rappelée) avec celui auquel on s'attend pour les vésicules unilamellaires et mesuré dans la même préparation de liposomes par microscopie électronique. Le compartiment intravésiculaire est mesuré en encapsulant soit des anticorps couplés à de la fluorescéine soit des ARN; on en déduit le diamètre suivant la relation connue entre les dimensions vésiculaires et le % de volume de substance encapsulée dans les vésicules unilamellaires.

Le tableau ci-dessus établit une nette correspondance entre les diamètres obtenus par ces deux techniques. Ainsi les liposomes de l'invention sont principalement unilamellaires.

En outre, ces liposomes renferment une quantité très importante de substance encapsulée, à savoir 35 l/mole de lipide dans le cas des liposomes PC-PC-cholestérol. Cette quantité est bien supérieure à celle que l'on peut obtenir par exemple par évaporation avec inversion de phase qui serait de 10 l/mole de lipide. De plus, une quantité raisonnable de lipide (13 mM) permet l'incorporation d'une proportion allant jusqu'à 50 % de macromolécules, ce qui est comparable aux résultats que l'on peut avoir dans le cas de l'inversion de phase. Par ailleurs, en raison du potentiel élevé créé dans de petits liposomes chargés, l'encapsulage de molécules portant la même charge est réduit. Ceci élimine la possibilité d'utiliser de petits liposomes contenant des PS pour encapsuler des acides nucléiques. Or, les liposomes de l'invention évitent cette contrainte et permettent le même taux d'encapsulage, aussi bien pour les ARN que pour les IgG qui sont des protéines légèrement positives.

Ainsi les liposomes de l'invention présentent toutes les propriétés recherchées pour le transfert de macromolécules à des cultures de cellules. De grosses vésicules sont plus efficaces pour des travaux portant sur le transfert de macromolécules à des cellules en raison du fait qu'elles peuvent encapsuler une quantité plus importante de substance par masse de lipides. C'est ainsi, par exemple, qu'un liposome de 1 000 nm véhicule $3.10^7$ molécules lorsqu'il est chargé avec une solution 1 M. De ce fait, la capture d'un liposome par cellule semble couvrir la plupart des cas. De plus, la composition de lipide (PS-PS-cholestérol) n'est pas toxique à l'égard de nombreuses cellules et permettrait même la fusion avec des membranes cellulaires sous des conditions connues pour favoriser ce phénomène comme, par exemple, l'utilisation de poly(éthylèneglycol). Dans ce cas, les liposomes unilamellaires de l'invention rendent le processus plus aisé.

Les IgG et ARN ne sont pas dénaturés et gardent toute leur activité après leur encapsulage. Le taux élevé d'incorporation (jusqu'à 50 % pour 13 mM de phospholipide) fait que l'invention convient très bien pour l'encapsulage de macromolécules qui sont normalement difficiles à obtenir en grandes quantités telles que les RNA, les anticorps monoclonés et les ADN monoclonés.

### 2.2. Préparation extemporanée:

Une application fort intéressante découlant de ces propriétés consiste à préparer et à administrer extemporanément un médicament à un patient en mélangeant directement la solution mixte (lipide + substance active + détergent) aux billes. Pour cela on procède de la façon suivante: Après avoir obtenu la solution mixte de lipides, de détergent et de la substance à administrer comme décrit ci-dessus, on procède à un prélèvement de cette solution au moyen d'une seringue munie à sa base d'un tamis et renfermant des billes telles que définies précédemment. Au bout de quelques minutes d'agitation, on peut, avec la même seringue, injecter les liposomes unilamellaires dépourvus de tout détergent et contenant la substance active résultant de cette opération conforme à l'invention avec un taux d'encapsulation compatible avec la quantité de lipides utilisée.

## Revendications

1. Procédé perfectionné pour l'obtention de liposomes unilamellaires de diamètres élevés supérieurs à 200 nm comprenant la solubilisation de lipides au moyen d'un détergent neutre, puis l'élimination dudit détergent par tout moyen approprié, procédé caractérisé par le fait que ledit détergent neutre présente une concentration critique micellaire élevée, qu'on utilise un rapport détergent: lipide de 10 : 1 à 12 : 1 et que l'élimination de ce détergent est effectuée au moyen de billes de polymères à pouvoir adsorbant élevé du type styrène-divinyl-benzène.

2. Procédé selon la revendication 1, caractérisé par le fait que détergent neutre est l'octylglucoside.

3. Procédé selon la revendication 1 ou 2, caractérisé par le fait que l'élimination du détergent se fait par contact direct avec lesdits polymères à pouvoir adsorbant élevé.

4. Procédé selon l'une quelconque des revendications 1 à 3, caractérisé par le fait que l'élimination du détergent se fait au moyen desdits polymères à pouvoir adsorbant élevé dans une opération de dialyse.

5. Application du procédé selon l'une quelconque des revendications 1 à 4 à l'obtention de liposomes unilamellaires de diamètres élevés allant de 200 à 1000 nm.

6. Application du procédé selon l'une quelconque des revendications 1 à 5 et des liposomes en résultant à l'encapsulage d'une substance active, caractérisée par le fait que l'on ajoute aux lipides solubilisés dans le détergent la substance à encapsuler et que l'on élimine ledit détergent au moyen desdits billes de polymères de synthèse à pouvoir adsorbant élevé.

7. Dispositif pour l'application selon la revendication 6, en vue de l'administration extemporanée d'un principe actif comprenant des moyens pour réaliser une solution mixte de lipides, de détergent et de la substance à administrer, des moyens pour prélever une quantité aliquote de cette solution, des moyens pour l'élimination dudit détergent par le procédé selon l'une quelconque des revendications 1 à 4 et des moyens d'administration aux patients.

## Patentansprüche

1. Verbessertes Verfahren zur Herstellung von unilamellaren Liposomen mit erhöhten Durchmessern von mehr als 200 nm, umfassend das Lösen van Lipiden mit einem neutralen Detergens und das anschließende Entfernen dieses Detergens durch ein geeignetes Mittel, welches Verfahren dadurch gekennzeichnet ist, daß das neutrale Detergens eine erhöhte kritische Mizellenkonzentration aufweist, ein Verhältnis Detergens: Lipid von 10 : 1 bis 12 : 1 verwendet wird und die Entfernung dieses Detergens mit Polymerkugeln mit erhöhtem Adsarptionsvermögen vom Styrol-Divinyl-Benzoltyp bewirkt wird.

2. Verfahren nach Anspruch 1, dadurch gekennzeichnet, daß das neutrale Detergens Octylglykosid ist.

3. Verfahren nach Anspruch 1 oder 2, dadurch gekennzeichnet, daß die Entfernung des Detergens durch direkte Berührung mit den Polymeren mit erhöhtem Adsorptionsvermögen erfolgt.

4. Verfahren nach einem der Ansprüche 1 bis 3, dadurch gekennzeichnet, daß die Entfernung des Detergens mit den genannten Polymeren mit erhöhtem Adsorptionsvermögen in einem Dialysevorgang erfolgt.

5. Verwendung des Verfahrens nach einem der Ansprüche 1 bis 4 zum Herstellen von unilamellaren Liposomen mit erhöhten Durchmessern von 200 bis 1000 nm.

6. Verwendung des Verfahrens nach einem der Ansprüche 1 bis 5 und von Liposomen zur Einkapselung einer aktiven Substanz, dadurch gekennzeichnet, daß man den im Detergens gelösten Lipiden die einzukapselnde Substanz zusetzt und das Detergens mit den genannten Synthesepolymeren mit erhöhtem Adsorptionsvermögen entfernt.

7. Vorrichtung zur Verwendung nach Anspruch 6 zur extemporierten Verabreichung eines Wirkstoffes, die Mittel zum Herstellen einer gemischten Lösung von Lipiden, Detergens und der zu verabreichenden Substanz, Mittel zum Entnehmen einer aliquoten Menge dieser Lösung, Mittel zum Entfernen des genannten Detergens durch das Verfahren nach einem der Ansprüche 1 bis 4 und Mittel zur Verabreichung an Patienten umfaßt.

## Claims

1. An improved process for obtaining unilamellar liposomes of large diameters greater than 200 nm comprising solubilization of lipids by means of a neutral detergent then elimination of the said detergent by any appropriate means, the process being characterized by the fact that the said neutral detergent has a high critical micellar concentration, a ratio of detergent to lipid of 10 : 1 to 12 : 1 is used, and the detergent is eliminated by means of balls of highly adsorbant polymers of the styrene-divinyl benzene kind.

2. A process according to claim 1, characterized in that the neutral detergent is octylglucoside.

3. A process according to claim 1 or 2, characterized by the fact that elimination of detergent is performed by direct contact with the said highly adsorbant polymers.

4. A process according to any one of claims 1 to 3, characterized by the fact that elimination of detergent is performed by means of the said highly adsorbant polymers in a dialysis operation.

5. Use of the process according to any one of claims 1 to 4 to obtain unilamellar liposomes having large diameters ranging from 200 to 1000 nm.

6. Use of the process according to any one of claims 1 to 5 and of the resultant liposomes for encapsulation of an active substance, characterized by the fact that the substance to be encapsulated is added to the lipids dissolved in the detergent and the detergent is eliminated by means of the said balls of highly adsorbant synthetic polymers.

7. A device for the use corresponding to claim 6 for extemporaneous administration of an active principle, comprising means to obtain a mixed solution of lipids, detergent and the substance to be administered, means for taking an aliquot quantity of this solution, means for eliminating the said detergent by a process according to any one of claims 1 to 4, and means for administration to patients.